# EUROPÄISCHE PATENTSCHRIFT

(11) **EP 2 780 708 B1**
(45) Veröffentlichungstag und Bekanntmachung des Hinweises auf die Patenterteilung: **16.01.2019**
(21) Anmeldenummer: 12821168.7
(22) Anmeldetag: 14.11.2012
(51) Int. Cl.: G01N 33/543, G01N 33/569

(54) **VERFAHREN ZUR ISOLIERUNG VON ZELLEN UND BIOPARTIKELN**
METHOD FOR ISOLATING CELLS AND BIOPARTICLES
PROCEDE D'ISOLEMENT DE CELLULES ET DE BIOPARTICULES

(30) Priorität: 14.11.2011 DE 102011118386
(43) Veröffentlichungstag der Anmeldung: 24.09.2014
(73) Patentinhaber: pluriSelect Life Sciences UG (haftungsbeschränkt) & Co. KG, 04103 Leipzig (DE)
(72) Erfinder: HEINRICH, Jan-Michael, 04275 Leipzig (DE); MOHR, Christoph, 04179 Leipzig (DE)
(74) Vertreter: Baumbach, Friedrich
(86) Internationale Anmeldenummer: PCT/DE2012/001106
(87) Internationale Veröffentlichungsnummer: WO 2013/071918

(56) Entgegenhaltungen:
- WO-A1-2011/034115
- WO-A2-2008/140573
- WO-A2-2009/005536

## Beschreibung

Die Erfindung beschreibt ein Verfahren zum zeitgleichen Isolieren von Zellen und/oder Biopartikeln aus komplexen Flüssigkeiten, die sich in einer oder mehreren Oberflächeneigenschaften unterscheiden. Mit ihr können unter Verwendung geeigneter Träger und bekannter Immobilisierungsverfahren unterschiedliche spezifische Zellen und/oder Biopartikel erkannt und schrittweise abgetrennt werden.
Die Erfindung beschreibt ein einfaches Verfahren zur schonenden Separierung/Isolierung von Zellen und/oder Partikeln aus Flüssigkeiten, vorzugsweise Blut. Dazu werden bekannte Schritte von Protein-Kopplungsverfahren, Protein-Spaltungsverfahren, Hydrolyse von intermolekularen und intramolekularen Wasserstoffbrücken, Protein Verdrängungsverfahren und Antigen-Antikörper-Ablösungsverfahren kombiniert mit dem Verfahren einer Fängerpartikel-Siebabtrennung.

Unter Zellen wird verstanden: Jeglicher somatische Zelltyp, unabhängig ob im physiologischen oder pathologischen Regulationszustand; Mikroorganismen wie Bakterien, Hefen/Pilze und Protozoen, sowie Viren. Unter Biopartikel ist jeglicher Zustand von organischen Molekülen verstanden, der mit dem Verlust der Löslichkeit in einem flüssigen Medium (z.B. Blut, Nährmedien, Zellkulturmedien, Zellaufbereitungen in Puffern, etc.) einhergeht. Zur Vereinfachung werden nachfolgend die Begriffe Zielzellen und Biopartikel unter dem Begriff "Targetpartikel" zusammengefasst.

Anwendungsgebiete der Erfindung sind Biotechnologie (einschließlich der biologischen und medizinischen Forschung), Diagnostik und die Behandlung von Krankheiten.

### Stand der Technik

Das Identifizieren, Separieren und Isolieren von Partikeln, insbesondere von somatischen Zellen und Krankheitserregern aus komplexen Flüssigkeiten wie Blut ist ein notwendiger Verfahrensschritt für die Erforschung, Diagnostik und Behandlung von Krankheiten.
Für die Separierung von Partikeln mit unterschiedlicher spezifischer Dichte (z.B. Leukozyten und Erythrozyten) haben sich Zentrifugationsverfahren oder Filter bewährt. Mit den Fortschritten in der Erforschung von Patho- und Immunogenese von Krankheiten besteht ein wachsendes Bedürfnis zur Identifizierung, Separierung und Isolierung von Zellen gleicher spezifischer Dichte aber unterschiedlicher Funktion. Die unterschiedliche Funktion von Lymphozyten z.B. wird durch die Expression von typischen Strukturen auf der äußeren Zellmembran begleitet (so z.B. die so genannten Cluster of Differentiation - CDs). Gegen diese Strukturen können Antikörper generiert werden, die das entscheidende Werkzeug für die mehr spezifischen Separationsverfahren sind. Fluoreszenz aktivierte Separationsverfahren und magnetische Trennverfahren haben sich seit Jahrzehnten für diese Aufgabe bewährt. Neuerdings wird durch die Firma pluriSelect (Deutschland) auch ein Fängerpartikel gestütztes Sieb-Sieb-Separationsverfahren angeboten (DE 10 2007 041 049.4). Diese Verfahren erlauben es auf einfache Weise, Zellen aus komplexen Flüssigkeiten zu isolieren, die sich in einem Merkmal auf der Membran von anderen unterscheiden.

Viele Zellen mit unterschiedlicher Funktion tragen aber häufig ein gleiches (überlappendes) neben differierenden Merkmalen, z.B. NK-Zellen (CD8+; CD56+, CD162R), zytotoxische T-Zellen (CD8+, CD3+), T-Helfer Zellen (CD3+; CD4+), regulatorische T-Zellen (CD3+, CD25+, CD4+).

Die Isolierung von Zellen, die nur eine gewünschte Kombination von CDs aufweist, ist nach wie vor eine große Herausforderung in der biologischen Forschung. Gegenwärtig kann nur mittels Fluorescence Activated Cell Sorting (FACS) nach Mehrfachmarkierungen diese Aufgabe gelöst werden. FACS ist zweifelsfrei der Goldstandard für die diagnostische Separation von Zellen, aber mit hohem apparativem und personellem Aufwand verbunden. Weitere Nachteile dieser Technik liegen in dem hohen Stress für die isolierten Zellen und es erfordert darüber hinaus ein kompliziertes steriles Arbeiten. Ferner sind auch der Sortierung einer größeren Anzahl vitaler Zellen methodische Grenzen gesetzt.
Probleme bereitet die FACS-Anwendung für die Isolierung großer Zellzahlen. Das die Zellen enthaltende Medium muss stark verdünnt werden, die Trennzeit für größere Zellmengen ist relativ lang und es bereitet Probleme, aseptische Bedingungen einzuhalten. Die Durchführung des Verfahrens verursacht insgesamt erhebliche Kosten und ist nicht für therapeutische (GMP konforme) Einsätze geeignet.

WO 2009/005536 A2 beschreibt den Einsatz von Mikropartikeln, an denen über Biotin-und Streptavidin-Linker Antikörper gebunden sind, die gegen mindestens 2 verschiedenen Antigene auf Bakterienzellen gerichtet sind. Allerdings ist es mit diesem System nicht möglich, zwei getrennte Gemische von einfach- und doppelt positiven Zielzellen zu isolieren.

Sowohl in WO 2008/140573 A2 als auch in WO 2011/034115 A1 wird die reversible Kopplung zwischen Desthiobiotin und Antikörpern beschrieben. Obwohl in diesen Dokumenten auf eine mögliche Verwendung dieser reversiblen Kopplung zur Seperation/Isolation von Zellen hingewiesen wird, ist es auch mit diesem Verfahren nicht möglich 2 getrennte Gemische von einfach- und doppelt positiven Zielzellen zu isolieren.

Eine weitere Erfindung zur Separation/Isolation von Zellen mit gleichen und unterschiedlichen Oberflächenstrukturen ist in DE 10 2009 037 331.4 beschrieben. Die Erfindung löst diese Aufgabenstellung durch eine neuartige Kombination von magnetischen und Fängerpartikel gestützten Sieb-Trennverfahren. Allerdings kommen in dieser Erfindung unterschiedliche Träger gleichzeitig zum Einsatz.

### Aufgabe / Ziel

Ziel der Erfindung ist es, ein einfaches Verfahren zur Separation komplexer Zellmischungen zu entwickeln, das es ermöglicht, mit wenigen Schritten kostengünstig Zellen und/oder Biopartikel abzutrennen, die sich in einer oder mehreren Oberflächeneigenschaften unterscheiden. Es soll auch für den Einsatz in komplexen Flüssigkeiten wie Blut oder Serum geeignet sein.

Die Erfindung wird gemäß den Ansprüchen 1 - 10 realisiert.

### Beschreibung der Erfindung

Gelöst wird die Aufgabe der Erfindung durch die Entwicklung eines *In vitro* Verfahrens, bei dem die Oberfläche eines festen Trägers in einer Art und Weise mit unterschiedlichen Fängermolekülen zu versehen wird, die es erlaubt, durch zusätzliche Verfahrensschritte gezielt einzelne Targetpartikel zu "neutralisieren". Dadurch sollen schrittweise unterschiedliche Targetpartikel mit sowohl gleichen wie auch unterschiedlichen Erkennungsstrukturen von den Fängerpartikeln abgelöst und isoliert/separiert werden.

Unter der Neutralisierung der Fängermolekül-Neutralisierung wird jegliche Behandlung verstanden, die eine Desintegration der Brücke zwischen Fängerpartikel und Target zur Folge hat, so z.B. durch dessen Ablösen/Abschneiden vom Fängerpartikel, oder durch Desintegration des Antikörper-Antigen-Bindungsortes. In deren Ergebnis besteht an diesem vorherigen Bindungsort nunmehr kein physischer Kontakt mehr zwischen Fänger-Partikel und Targetpartikel.
Die unterschiedlichen Strategien können je nach Zielstellung kombiniert werden.

Die Erfindung betrifft insbesondere ein Verfahren zur Separierung und Isolierung von unterschiedlichen Zell- oder Biopartikeln mittels eines festen Trägers, dadurch gekennzeichnet, dass der feste Träger, auf dessen Oberfläche wenigstens zwei Fängermoleküle unterschiedlicher Target-Spezifität mit bekannten Methoden gekoppelt sind und von dem wenigstens ein Fängermolekül mittels bekannter Verfahren so behandelt werden kann, dass die direkte Brücken-Verbindung zwischen Fängermolekül und Targetpartikeln, oder zwischen Fängermolekül und Träger aufgelöst wird, mit einer die Targetpartikel enthaltenden Flüssigkeit in Kontakt gebracht wird und anschließend die Träger-Fängermolekül-Target-Komplexe erst mittels bekannter Verfahren isoliert und dann die Targetpartikel über eine Spaltung der jeweiligen Brücken-Verbindung sequenziell abgelöst werden.

Besonders geeignet ist es dabei, wenn als Fängermoleküle Antikörper verwendet werden, die auf die Träger-Oberfläche adsorptiv oder kovalent oder nicht kovalent gebunden sind.

Besonders vorteilhaft ist es, wenn die Fängermoleküle direkt oder über Spacer/Brücken-Moleküle mit vorbestimmten Eigenschaften für eine chemische oder enzymatische oder physikalische Trennung von Spacer und Target versehen sind werden und wenn die Fängermoleküle vorzugsweise zusätzlich magnetisierbar und/oder fluoreszierbar gemacht werden.

Vorteilhaft ist die Verwendung eines Verfahrens, bei dem die die Brücken-Verbindung zwischen dem Träger-Fängermolekül-Komplex und dem Targetpartikel aufgelöst wird durch eine Trennung des Fängermoleküls vom Targetpartikel oder vom Träger oder durch Auflösung der Bindung zwischen Fängermolekül und Targetpartikel.

Besonders geeignet ist ein Verfahren, bei dem die Trennung des Fängermoleküls vom Targetpartikel oder vom festen Träger durch die Einwirkung von Enzymen oder Wasserstoffbrückenbindung reduzierenden Substanzen erfolgt.

Insbesondere die Auflösung der Bindung zwischen Fängermolekül und Targetpartikel durch eine Verdrängungsreaktion durch Rezeptor- oder Akzeptor-imitierende Substanzen mit höherer spezifischer Bindungsstärke als die zwischen Fängermolekülrezeptor und Targetpartikel-Akzeptor hat sich bewährt.

Vorzugweise erfolgt dabei für jede gewünschte Spezifität von Fängermolekül auf dem festen Träger eine unterschiedliche Prozedur der Auflösung der direkten Brücken-Verbindung zwischen dem Träger-Fängermolekül-Komplex und Targetpartikeln vorgesehen wird.

In einer vorteilhaften Ausführungsform werden unbegrenzt viele unterschiedliche Fängermolekül-Spezifitäten auf der Oberfläche eines festen Trägers gebunden, limitiert lediglich durch sterische Behinderung, durch die Anzahl von Targetpartikeln und die Summe der zur Verfügung stehenden Verfahren zur Auflösung der direkten Brücken-Verbindung zwischen Träger-Fängermolekül-Komplex und Targetpartikel.

In einer weiteren vorteilhaften Ausführungsform werden für die Auflösung der Brücken-Verbindung zwischen festem Träger-Fängermolekül-Komplex und Targetpartikel beispielsweise reduzierende Agentien wie β-Mercaptoethanol, Dithiothreitol, Dithioerythritol, Tris(hydroxypropyl)phosphine, Tris(2-carboxyethyl)phosphine, Enzyme je nach der vorhandenen Schnittstelle innerhalb oder in der Umgebung des Spacers und Substanzen mit höherer Avidität als die vorhandene zwischen Fängermolekülrezeptor und Targetpartikelakzeptor wie z.B. das Verdrängen von Destiobiotin durch Biotin verwendet.

in einer weiteren Ausbildung der Erfindung werden die mittels festem Träger separierten Targetpartikel mit sowohl übereinstimmenden wie abweichenden Fängermolekül-Targetspezifitäten durch schrittweise Auflösung der Brücken-Verbindung zwischen Träger-Fängermolekül-Komplex und Targetpartikel und nachfolgender Abtrennung mittels bekannter magnetischer, gravimetrischer oder Sieb-Verfahren in jeweils homogene Targetpopulationen mit gleicher Targetrezeptor/Akzeptor-Spezifität separiert und isoliert.

Ein anderer Aspekt der Erfindung betrifft die Verwendung des erfindungsgemäßen festen Trägers, wobei der feste Träger jegliche geometrische Form haben kann, vorzugsweise Mikropartikel. Besonders bevorzugt sind Trägermaterialien aus Polystyrol (PS), Polymethacrylat (PMA), Polyaktide (PLA) oder anderen Kunststoffen. Ebenso besonders geeignet sind Trägermaterialien aus Sepharosen, Alginate oder ähnliche organische Materialien.

Insbesondere geeignet ist das erfindungsgemäße Verfahren für die Separierung von Zellen aus Körperflüssigkeiten mit Anwendung in der Biotechnologie, medizinischen Forschung, Diagnostik und Therapie von Krankheiten

Während die sequenzielle Separation von Zellen mit gleichen und unterschiedlichen Oberflächenstrukturen Stand der Technik ist, erlaubt das erfindungsgemäße Verfahren unter der Verwendung von nur einem festen Träger mit wenigstens zwei Fängermolekülen unterschiedlicher Target-Spezifität erstmals eine echte zeitgleiche Abtrennung von Zellen und/oder Biopartikeln. Das Verfahren kann mit großen Zellzahlen, geringem Aufwand und wenig Arbeitsmaterial in kurzer Zeit und stressarm für die Zellen durchgeführt werden. Das Verfahren erlaubt die gleichzeitige Isolierung/Separierung von sub-Populationen mit überlappenden Oberflächeneigenschaften mit Hilfe von einfachen physikalischen Trennmethoden. Das Verfahren ermöglicht den direkten Einsatz in einem Reaktor im Vollblut an einem Probanden und ist damit therapeutisch/diagnostisch extrakorporal einsetzbar.

Die nachfolgenden Ausführungsbeispiele beschreiben die methodische Umsetzung für einzelne Anwendungen. Der Fachmann kann die Anwendungen entsprechend der jeweiligen Zielstellung beliebig kombinieren und modifizieren.

### Beispiel 1

### Kombination von Fängerneutralisierung durch Ablösung des Fänger-Target-Komplexes vom Fängerpartikel mit nicht neutralisierbaren Fängermolekülen

An Fängerpartikel (z.B. Polystyrene Partikel mit 30µm Durchmesser) wird anti-CD8 Antikörper kovalent über Carboxylgruppen (NHS/EDS) und anti-CD3 Antikörper über einen Destiobiotin Linker gekoppelt. Die Fängerpartikel werden in Vollblut verbracht und für 15 min rollend inkubiert. Während dieser Zeit binden CD3 - positive und CD8 - positive Zellen an dem Partikel. Diese werden mittels eines passenden Siebes (z.B. pluriStrainer) aus dem Vollblut isoliert und gewaschen. Danach wird der Destiobiotin Linker gekoppelte anti-CD3 Antikörper über die Zugabe von 12mM Biotin Lösung vom Fängerpartikel verdrängt. Am Partikel verbleiben alle CD8 - positiven Zellen inklusive der CD3/CD8 - positiven Zellen. Abgelöste Zellen sind CD3+ und CD8-.

### Beispiel 2

### Kombination von Fängerneutralisierung durch Desintegration des Fänger/Antikörper-Target/Antigen-Komplexes mit nicht neutralisierbaren Fängermolekülen

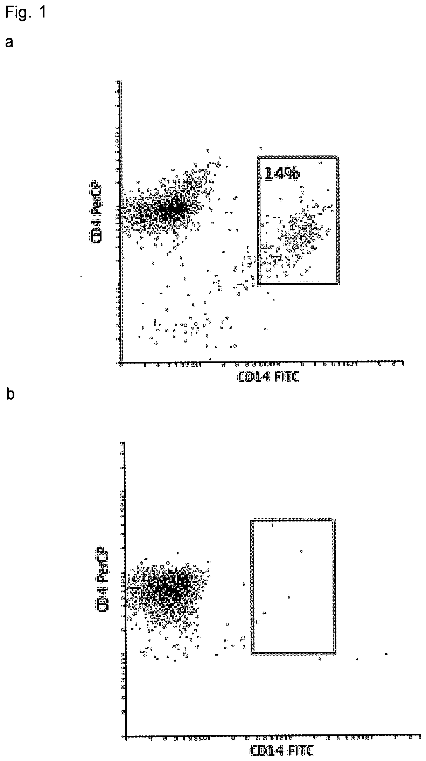

An Fängerpartikel (z.B. Polystyrene Partikel mit 30µm Durchmesser) wird anti-CD14 Antikörper kovalent über Carboxylgruppen (NHS/EDS) und anti-CD4 Antikörper über einen Linker gekoppelt. Die Fängerpartikel werden in Vollblut verbracht und für 15 min rollend inkubiert. Während dieser Zeit binden CD14 - positive und CD4 - positive Zellen an dem Partikel. Diese werden mittels eines passenden Siebes (z.B. pluriStrainer) aus dem Vollblut isoliert und gewaschen. Danach wird zum anti-CD4 Antikörper höher affines Peptid dazugegeben, dieses verdrängt den Antikörper vom Rezeptor auf der Zelle. Am Partikel verbleiben alle CD14 - positive Zellen inklusive der CD4/CD14 - positiven Zellen. Abgelöste Zellen sind CD4+ und CD14- (Figur 1).

### Beispiel 3

### Kombination von Fängerneutralisierung durch Desintegration und durch Ablösung mit nichtneutralisierbaren Fänger-Molekülen

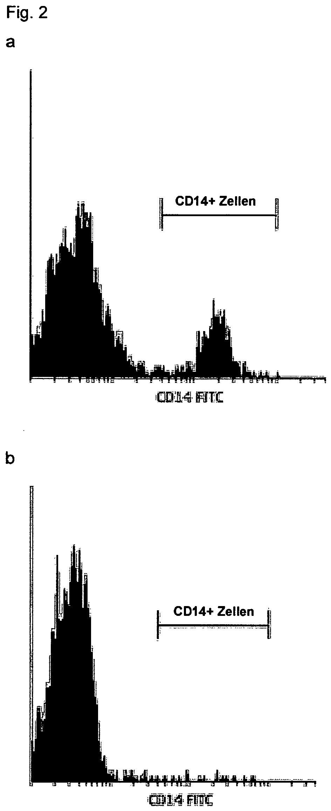

An Fängerpartikel (z.B. Polystyrene Partikel mit 30µm Durchmesser) wird anti-CD14 Antikörper kovalent über Carboxylgruppen (NHS/EDS) und anti-CD4 Antikörper über einen DNA Linker gekoppelt. Die Fängerpartikel werden in Vollblut verbracht und für 15 min rollend inkubiert. Während dieser Zeit binden CD14 - positive und CD4 - positive Zellen an dem Partikel. Diese werden mittels eines passenden Siebes (z.B. pluriStrainer) aus dem Vollblut isoliert und gewaschen. Danach wird der DNA Linker gekoppelte anti-CD4 Antikörper über die Zugabe von einer DNAse Lösung vom Fängerpartikel abgeschnitten. Am Partikel verbleiben alle CD14 - positive Zellen inklusive der CD4/CD14 - positiven Zellen. Abgelöste Zellen sind CD4+ und CD14-(Figur 2).

### Beispiel 4

### Kombination von Fängerneutralisierung durch Desintegration und durch Ablösung des Fänger-Target-Komplexes vom Fängerpartikel

An Fängerpartikel (z.B. Polystyrene Partikel mit 30µm Durchmesser) wird anti-CD3 Antikörper über einen Destiobiotin Linker gekoppelt und anti-CD4 Antikörper über einen DNA - Linker gekoppelt. Die Fängerpartikel werden in Vollblut verbracht und für 15 min rollend inkubiert. Während dieser Zeit binden CD3 - positive und CD4 - positive Zellen an dem Partikel. Diese werden mittels eines passenden Siebes (z.B. pluriStrainer) aus dem Vollblut isoliert und gewaschen. Danach wird zuerst wird der Destiobiotin Linker gekoppelte anti-CD3 - Antikörper über die Zugabe von 12mM Biotin Lösung vom Fängerpartikel verdrängt. Am Partikel verbleiben alle CD4 - positiven Zellen inklusive der CD4/CD3 - positiven Zellen. Abgelöste Zellen sind CD3+ und CD4-. Danach wird der DNA Linker gekoppelte anti-CD4 - Antikörper über die Zugabe von einer DNAse Lösung vom Fängerpartikel abgeschnitten. Abgelöste Zellen sind CD3+ und CD4+.

### Beispiel 5

### Kombination von Fängerneutralisierung durch Desintegration und durch Ablösung des Fänger-Target-Komplexes vom Fängerpartikel und vom Fängerpartikel mit nicht neutralisierbaren Fängermolekülen

An Fängerpartikel (z.B. Polystyrene Partikel mit 30µm Durchmesser) wird anti-CD14 Antikörper kovalent über Carboxylgruppen (NHS/EDS) und anti-CD3 Antikörper über einen Destiobiotin Linker gekoppelt und anti-CD4 Antikörper über einen DNA - Linker gekoppelt. Die Fängerpartikel werden in Vollblut verbracht und für 15 min rollend inkubiert. Während dieser Zeit binden CD3 - positive und CD4 - positive Zellen und CD14 - positive Zellen an dem Partikel. Diese werden mittels eines passenden Siebes (z.B. pluriStrainer) aus dem Vollblut isoliert und gewaschen. Danach wird zuerst wird der Destiobiotin Linker gekoppelte anti-CD3 - Antikörper über die Zugabe von 12mM Biotin Lösung vom Fängerpartikel verdrängt. Am Partikel verbleiben alle CD4 - und CD14 - positiven Zellen inklusive der CD4/CD3 - und CD4/CD14 - positiven Zellen. Abgelöste Zellen sind CD3+ und CD4- und CD14-. Danach wird der DNA Linker gekoppelte anti-CD4 Antikörper über die Zugabe von einer DNAse Lösung vom Fängerpartikel abgeschnitten. Abgelöste Zellen sind CD3+ und CD4+ und CD14-. Am Partikel verbleiben alle CD14 - positive Zellen inklusive der CD4/CD14 - positiven Zellen.

### Beschreibung der Figuren

- Figur 1a:: Isolation von CD4 - positiven Zellen aus Vollblut mit Ablösung durch Verdrängung ohne Kombination mit kovalent gebundenen CD14.
- Figur 1b:: Abbildung 2 Isolation von CD4 - positiven Zellen aus Vollblut mit Ablösung durch Verdrängung mit der Kombination mit kovalent gebundenen CD14
- Figur 2a:: Isolation von CD4 - positiven Zellen aus Vollblut mit Ablösung durch DNAse - Verdau der DNA Linkers ohne Kombination mit kovalent gebundenen CD14.
- Figur 2b:: Isolation von CD4 - positiven Zellen aus Vollblut mit Ablösung durch DNAse - Verdau der DNA Linkers mit der Kombination mit kovalent gebundenen CD14.

## Patentansprüche

1. *In vitro* Verfahren zur Separierung und Isolierung von unterschiedlichen Zellen mittels Mikropartikeln, **dadurch gekennzeichnet, dass** Mikropartikel verwendet werden, an deren Oberfläche wenigstens zwei Antikörper unterschiedlicher Zielzell-Spezifität adsorptiv oder kovalent oder nicht kovalent gebunden werden, wobei die Antikörper mittels bekannter Verfahren so behandelt werden, dass für jede Zielzell-Spezifität eines Antikörpers eine unterschiedliche Prozedur der Auflösung der direkten Brücken-Verbindung zwischen Antikörper und Zielzelle, oder zwischen Antikörper und Mikropartikel ermöglicht wird, und die Mikropartikel-Antikörper-Komplexe mit einer die Zielzellen enthaltenden Flüssigkeit in Kontakt gebracht werden und anschließend die Mikropartikel-Antikörper-Zielzell-Komplexe erst mittels bekannter Verfahren isoliert und dann die Zielzellen über eine Spaltung der jeweiligen Brücken-Verbindung sequenziell abgelöst werden.

2. Verfahren nach Anspruch 1, **dadurch gekennzeichnet, dass** die Antikörper mit Spacer/Brücken-Molekülen gekoppelt sind, wobei die Spacer/Brücken-Moleküle mit vorbestimmten Eigenschaften für eine chemische oder enzymatische oder physikalische Trennung von Antikörper und Mikropartikel oder Zielzelle versehen sind.

3. Verfahren nach Anspruch 1-2, **dadurch gekennzeichnet, dass** die Antikörper zusätzlich magnetisierbar und/oder fluoreszierbar gemacht sind.

4. Verfahren nach Anspruch 1-3 **dadurch gekennzeichnet, dass** die Bindung zwischen dem Mikropartikel-Antikörper-Komplex und der Zielzelle aufgelöst wird durch eine Trennung des Antikörpers von der Zielzelle oder von den Mikropartikeln oder durch Auflösung der Bindung zwischen Antikörper und Zielzelle.

5. Verfahren nach Anspruch 1-4, **dadurch gekennzeichnet, dass** die Trennung des Antikörpers von der Zielzelle oder von den Mikropartikeln durch die Einwirkung von Enzymen oder Wasserstoffbrückenbindung reduzierenden Substanzen erfolgt.

6. Verfahren nach Anspruch 1-5, **dadurch gekennzeichnet, dass** die Auflösung der Bindung zwischen Antikörper und Zielzelle durch eine Verdrängungsreaktion durch Rezeptor- oder Akzeptor-imitierende Substanzen mit höherer spezifischer Bindungsstärke als die zwischen Antikörperrezeptor und Zielzellen-Akzeptor erfolgt.

7. Verfahren nach Anspruch 1-6, **dadurch gekennzeichnet, dass** unbegrenzt viele unterschiedliche Antikörper-Spezifitäten auf der Oberfläche der Mikropartikel gebunden werden, limitiert lediglich durch sterische Behinderung, durch die Anzahl von Zielzellen und die Summe der zur Verfügung stehenden Verfahren zur Auflösung der Bindung zwischen Mikropartikel-Antikörper-Komplex und Zielzelle.

8. Verfahren nach Anspruch 7, **dadurch gekennzeichnet, dass** für die Auflösung der Bindung zwischen Mikropartikel-Antikörper-Komplex und Zielzelle reduzierende Agentien wie β-Mercaptoethanol, Dithiothreitol, Dithioerythritol; Tris(hydroxypropyl)phosphine, Tris(2-carboxyethyl)phosphine, Enzyme je nach der vorhandenen Schnittstelle innerhalb oder in der Umgebung des Spacers und Substanzen mit höherer Avidität als die vorhandene zwischen Fängermolekülrezeptor und Targetpartikelakzeptor wie z.B. das Verdrängen von Destiobiotin durch Biotin verwendet werden.

9. Verfahren nach Anspruch 1-8, **dadurch gekennzeichnet, dass** die mittels Mikropartikel separierten Zielzellen mit sowohl übereinstimmenden wie abweichenden Antikörper-Zielzellspezifitäten durch schrittweise Auflösung der Bindung zwischen Mikropartikel-Antikörper-Komplex und Zielzelle und nachfolgender Abtrennung mittels bekannter magnetischer, gravimetrischer oder Sieb-Verfahren in jeweils homogene Zielzellpopulationen mit gleicher Zielzellrezeptor/Akzeptor-Spezifität separiert und isoliert werden.

10. Verwendung eines Verfahrens nach Anspruch 1-9, für die Separierung von Zellen aus Körperflüssigkeiten mit Anwendung in der Biotechnologie, Medizinischen Forschung, Diagnostik und Therapie von Krankheiten.

## Claims

1. *In vitro* method for separating and isolating various cells by means of microparticles, **characterized in that** microparticles are used on the surface of which at least two antibodies of different target cell specificity are bound adsorptively or covalently or non-covalently, wherein the antibodies are being treated by known methods such that for each target cell specificity of an antibody a different procedure for dissolving the direct bridge connection between the antibody and the target cell, or between the antibody and the microparticles is provided, and the microparticle-antibody complexes are brought into contact with a liquid containing the target cells and subsequently the microparticle-antibody-target cell complexes are first isolated by known methods and then the target cells are detached sequentially by cleaving the respective bridge connection.

2. Method according to claim 1, **characterized in that** the antibodies are coupled to spacer/bridge molecules, wherein the spacer/bridge molecules are provided with predetermined properties for a chemical or enzymatic or physical separation of antibody and microparticles or target cell.

3. Method according to claim 1-2, **characterized in that** the antibodies are made additionally magnetisable and/or fluorescent.

4. Method according to claim 1-3, **characterized in that** the binding between the microparticle-antibody complex and the target cell is dissolved by separating the antibody from the target cell or from the microparticles or by dissolving the binding between antibody and target cell.

5. Method according to claim 1-4, **characterized in that** the separation of the antibody from the target cell or from the microparticles takes place by the action of enzymes or hydrogen bond reducing substances.

6. Method according to claim 1-5, **characterized in that** the binding between antibody and target cell is dissolved by a displacement reaction by receptor- or acceptor-mimicking substances with higher specific binding strength than that between antibody receptor and target cell acceptor.

7. Method according to claim 1-6, **characterized in that** an unlimited number of different antibody specificities are bound on the surface of the microparticles, limited only by steric hindrance, by the number of target cells and by the sum of the available methods for dissolving the binding between microparticle-antibody complex and target cell.

8. Method according to claim 7, **characterized in that** for dissolving the bond between microparticle-antibody complex and target cell, reducing agents such as β-mercaptoethanol, dithiothreitol, dithioerythritol; tris (hydroxypropyl) phosphine, tris (2-carboxyethyl) phosphine, enzymes depending on the existing interface within or in the vicinity of the spacer and substances with a higher avidity than those existing between the capture molecule receptor and target particle acceptor are used, such as the displacement of distiobiotin by biotin.

9. Method according to claim 1-8, **characterized in that** with the use of microparticles separated target cells having both matching and not matching antibody target cell specificities are separated and isolated by gradual dissolution of the binding between microparticle-antibody complex and target cell followed by a subsequent separation by means of known magnetic, gravimetric or sieving methods into respective homogeneous target cell populations having the same target cell receptor/acceptor specificity.

10. Use of a method according to claim 1-9, for the separation of cells from body fluids with applications in biotechnology, medical research, diagnostics and therapy of diseases.

## Revendications

1. Procédé *In vitro* pour la séparation et l'isolation de différentes cellules au moyen de microparticules, **caractérisé par** l'utilisation de microparticules à la surface desquelles au moins deux anticorps avec des spécificités de cellules cibles différentes sont liés par adsorption, de manière covalente ou non covalente, les anticorps étant traités au moyen de procédés connus, de façon à permettre, pour chaque spécificité de cellule cible d'un anticorps, une procédure différente de dissolution de la liaison en pont directe entre l'anticorps et la cellule cible ou entre l'anticorps et la microparticule et à ce que les complexes microparticule-anticorps soient mis en contact avec un liquide contenant les cellules cibles, puis à ce que les complexes microparticule-anticorps-cellule cible soient isolés au moyen de procédés connus et que les cellules cibles soient dissoutes séquentiellement par une scission de la liaison en pont respective.

2. Procédé conformément à la revendication n°1, **caractérisé par le fait que** les anticorps sont couplés à des molécules espaceur/pont, les molécules espaceur/pont étant dotées de propriétés prédéterminées pour une séparation chimique, enzymatique ou physique de l'anticorps et de la microparticule ou de la cellule cible.

3. Procédé conformément aux revendications n°1 à n°2, **caractérisé par le fait que** les anticorps sont en plus rendus magnétisables et/ou potentiellement fluorescents.

4. Procédé conformément aux revendications n°1 à n°3, **caractérisé par le fait que** la liaison entre le complexe microparticule-anticorps et la cellule cible est dissoute par une séparation de l'anticorps de la cellule cible ou des microparticules ou par une dissolution de la liaison entre l'anticorps et la cellule cible.

5. Procédé conformément aux revendications n°1 à n°4, **caractérisé par le fait que** la séparation de l'anticorps de la cellule cible ou des microparticules s'opère par l'action d'enzymes ou de substances réduisant la liaison hydrogène.

6. Procédé conformément aux revendications n°1 à n°5, **caractérisé par le fait que** la dissolution de la liaison entre l'anticorps et la cellule cible s'opère par une réaction de déplacement par des substances imitant les récepteurs ou les accepteurs, ayant une force de liaison spécifique plus élevée que celle entre le récepteur de l'anticorps et l'accepteur de la cellule cible.

7. Procédé conformément aux revendications n°1 à n°6, **caractérisé par le fait qu'**un nombre illimité de spécificités d'anticorps différentes est lié à la surface des microparticules, restreint uniquement par un empêchement stérique, par le nombre de cellules cibles et le total des procédés disponibles pour la dissolution de la liaison entre le complexe microparticule-anticorps et la cellule cible.

8. Procédé conformément à la revendication n°7, **caractérisé par le fait que**, pour la dissolution de la liaison entre le complexe microparticule-anticorps et les agents réduisant la cellule cible, comme le β-mercaptoéthanol, le dithiothréitol, le dithioérythritol, la tris(hydroxypropyl)phosphine, la tris(2-carboxyéthyl)phosphine, des enzymes, selon la jonction existante à l'intérieur ou dans l'environnement de l'espaceur, et des substances avec une avidité plus élevée que celle qui existe entre le récepteur de la molécule de capture et l'accepteur de la particule cible, comme par exemple le déplacement de destiobiotine par la biotine, sont utilisées.

9. Procédé conformément aux revendications n°1 à n°8, **caractérisé par le fait que** les cellules cibles séparées au moyen de microparticules, avec des spécificités anticorps-cellule cible convergentes ou divergentes, sont séparées et isolées par une dissolution progressive de la liaison entre le complexe microparticule-anticorps et la cellule cible et par une séparation consécutive au moyen de procédés magnétiques, gravimétriques ou de criblage connus dans des populations de cellules cibles respectivement homogènes, ayant la même spécificité de récepteur/accepteur de cellule cible.

10. Utilisation d'un procédé conformément aux revendications n°1 à n°9, pour la séparation de cellules de liquides corporels avec application en biotechnologie, recherche médicale, diagnostic et traitement de maladies.
